(19)

**Europäisches Patentamt**
**European Patent Office**
**Office européen des brevets**

(11) **EP 4 718 461 A1**

(12) **EUROPEAN PATENT APPLICATION**
published in accordance with Art. 153(4) EPC

(43) Date of publication:
**01.04.2026 Bulletin 2026/14**

(21) Application number: **24822215.0**

(22) Date of filing: **24.01.2024**

(51) International Patent Classification (IPC):
**G16B 40/00** (2019.01)    **G06F 18/213** (2023.01)
**G06F 16/9035** (2019.01)

(52) Cooperative Patent Classification (CPC):
**G06F 16/9035; G06F 18/213; G16B 40/00**

(86) International application number:
**PCT/CN2024/073782**

(87) International publication number:
**WO 2024/255254 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.06.2023   CN 202310727647**

(71) Applicant: **Huawei Technologies Co., Ltd.
Shenzhen, Guangdong 518129 (CN)**

(72) Inventors:
• **YOU, Peiting
  Shenzhen, Guangdong 518129 (CN)**
• **YU, Chen
  Shenzhen, Guangdong 518129 (CN)**
• **FAN, Yuwei
  Shenzhen, Guangdong 518129 (CN)**
• **ZHU, Chuan
  Shenzhen, Guangdong 518129 (CN)**

(74) Representative: **Goddar, Heinz J.
Boehmert & Boehmert
Anwaltspartnerschaft mbB
Pettenkoferstrasse 22
80336 München (DE)**

(54) **DATA PROCESSING METHOD AND APPARATUS**

(57)    Embodiments of this application relate to the field of data processing technologies, and disclose a data processing method and apparatus, to select a core SNP with a high heritability characteristic from biological SNPs. The method includes: obtaining a first SNP set; determining a second SNP set from the first SNP set based on first information of an SNP; and determining a third SNP set from the second SNP set based on a target feature extraction method and a non-linear feature extraction method. The first SNP set includes N SNPs, N is a positive integer, the first information includes at least one of linkage disequilibrium information or a variance, the second SNP set includes M SNPs, M is a positive integer, M is less than N, the target feature extraction method includes at least one of a filter feature extraction method, an envelope feature extraction method, or an embedded feature extraction method, the third SNP set includes H SNPs, H is a positive integer, and H is less than M.

FIG. 2

## Description

This application claims priority to Chinese Patent Application No. 202310727647.3, filed with the China National Intellectual Property Administration on June 16, 2023 and entitled "DATA PROCESSING METHOD AND APPARATUS", which is incorporated herein by reference in its entirety.

## TECHNICAL FIELD

Embodiments of this application relate to the field of data processing technologies, and in particular, to a data processing method and apparatus.

## BACKGROUND

A single nucleotide polymorphism (single nucleotide polymorphism, SNP) is mainly a DNA sequence polymorphism caused by a variation of a single nucleotide at a genome level. The SNP, as one of most common biological genetic variations, accounts for more than 90% of all known polymorphisms. The SNP is widely present in biological genomes, occurring on average once every 300 base pairs, with an estimated total quantity of 3 million or even more. The SNP is a biallelic marker, and is caused by transition or transversion of a single base, or insertion or absence of a base. The SNP may be in a gene sequence, or may be in a non-coding sequence outside a gene. During gene selection, a core SNP with high heritability needs to be selected from biological SNPs.

However, only a few of tens of thousands of biological SNPs are core SNPs with a high heritability characteristic.

Therefore, how to select the core SNP with the high heritability characteristic from the biological SNPs is one of problems that need to be urgently resolved by a person skilled in the art.

## SUMMARY

Embodiments of this application relate to the field of data processing technologies, and provide a data processing method and apparatus, to select a core SNP with a high heritability characteristic from biological SNPs. To achieve the foregoing objectives, the following technical solutions are used in embodiments of this application.

According to a first aspect, an embodiment of this application provides a data processing method. The method includes: obtaining a first SNP set; determining a second SNP set from the first SNP set based on first information of an SNP; and determining a third SNP set from the second SNP set based on a target feature extraction method and a non-linear feature extraction method. The first SNP set includes N SNPs, N is a positive integer, the first information includes at least one of linkage disequilibrium information or a variance, the second SNP set includes M SNPs, M is a positive integer, M is less than N, the target feature extraction method includes at least one of a filter feature extraction method, an envelope feature extraction method, or an embedded feature extraction method, the third SNP set includes H SNPs, H is a positive integer, and H is less than M.

It should be noted that one SNP in a plurality of SNPs in one region may represent types of most SNPs in the region. In this embodiment of this application, a representative SNP in each region is determined from a plurality of obtained SNPs based on a variance and linkage disequilibrium information of the SNP, and the other remaining redundant SNPs are removed. Therefore, data dimension reduction is performed on obtained SNP data, and then feature selection is performed on the dimension-reduced data by using the target feature extraction method and the non-linear feature extraction method, to select a core SNP with a high heritability characteristic from biological SNPs. In addition, in this embodiment of this application, an SNP that is strongly correlated with a dependent variable (phenotype) is also selected from the plurality of obtained SNPs based on the variance and the linkage disequilibrium information of the SNP.

Optionally, the linkage disequilibrium information may include a linkage disequilibrium (linkage disequilibrium, LD) coefficient.

In a possible implementation, a target SNP may be deleted from the first SNP set based on the LD coefficient of the SNP to obtain the second SNP set, where the target SNP is an SNP whose LD coefficient is outside a preset LD coefficient interval.

It may be understood that, if the LD coefficient of the SNP is outside the preset LD coefficient interval, there is a high probability that the SNP is in a linkage disequilibrium state, and the SNP in the linkage disequilibrium state is likely to have a same genetic characteristic as another SNP. Therefore, the target SNP of the obtained SNP data may be removed, to perform data dimension reduction on the obtained SNP data, and then feature selection is performed on the dimension-reduced data by using the target feature extraction method and the non-linear feature extraction method, to select the core SNP with the high heritability characteristic from the biological SNPs.

In a possible implementation, when there are a plurality of SNPs with a same variance in the first SNP set, one SNP in the plurality of SNPs with the same variance is retained, and the other SNPs in the plurality of SNPs are deleted to obtain the second SNP set.

It may be understood that the SNPs with the same variance may also have a same genetic characteristic. Therefore, only the one SNP in the plurality of SNPs with the same variance may be retained, and the other SNPs in the plurality of SNPs with the same variance are deleted from the obtained SNP data, to perform data dimension reduction on the obtained SNP data, and then feature selection is performed on the dimension-reduced data by using the target feature extraction method and the non-linear feature extraction method, to select the core SNP with the high heritability characteristic from the biological SNPs.

In a possible implementation, a fourth SNP set may be determined from the second SNP set based on the target feature extraction method, where the fourth SNP set includes K SNPs, K is a positive integer, and K is less than M and greater than H. The third SNP set is determined from the fourth SNP set based on the non-linear feature extraction method.

It can be learned that, in this embodiment of this application, the representative SNP in each region is determined from the plurality of obtained SNPs based on the variance and the linkage disequilibrium information of the SNP, and the remaining redundant SNPs are removed. Therefore, data dimension reduction is performed on the obtained SNP data, and then feature selection is separately performed on the dimension-reduced data by using the target feature extraction method and the non-linear feature extraction method, to select the core SNP with the high heritability characteristic from the biological SNPs.

In a possible implementation, an SNP may be removed from the second SNP set based on the target feature extraction method. When a coefficient of determination of remaining SNPs in the second SNP set is greater than a first threshold or a first remaining ratio is less than a first ratio threshold, the remaining SNPs in the second SNP set are determined as the fourth SNP set, where the first remaining ratio is a ratio of a quantity of the remaining SNPs in the second SNP set to M.

It can be learned that, according to the method provided in this embodiment of this application, the representative SNP in each region is determined from the plurality of obtained SNPs based on the variance and the linkage disequilibrium information of the SNP, and the remaining redundant SNPs are removed. Therefore, data dimension reduction is performed on the obtained SNP data, then selection is further performed on the plurality of obtained SNPs by using the target feature extraction method based on the coefficient of determination and a quantity of the remaining SNPs, and feature selection is performed, by using a non-linear feature, on the SNPs obtained through further selection, to select the core SNP with the high heritability characteristic from the biological SNPs. In addition, selection is further performed on the plurality of obtained SNPs by using the target feature extraction method based on the coefficient of determination and the quantity of the remaining SNPs, so that an SNP of a penalty term of a correlation between features can be obtained.

In a possible implementation, an SNP may be removed from the fourth SNP set based on the non-linear feature extraction method. When a coefficient of determination of remaining SNPs in the fourth SNP set is greater than a second threshold or a second remaining ratio is less than a second ratio threshold, the remaining SNPs in the fourth SNP set are determined as the third SNP set, where the second remaining ratio is a ratio of a quantity of the remaining SNPs in the fourth SNP set to M.

It can be learned that, according to the method provided in this embodiment of this application, the representative SNP in each region is determined from the plurality of obtained SNPs based on the variance and the linkage disequilibrium information of the SNP, and the remaining redundant SNPs are removed. Therefore, data dimension reduction is performed on the obtained SNP data, then selection is further performed on the plurality of obtained SNPs by using the target feature extraction method, and selection is performed again, from the plurality of obtained SNPs based on the coefficient of determination and a quantity of the remaining SNPs by using a non-linear feature, on the SNPs obtained through further selection, to select the core SNP with the high heritability characteristic from the biological SNPs.

According to a second aspect, an embodiment of this application provides a data processing apparatus. The data processing apparatus includes a transceiver unit and a processing unit. The transceiver unit is configured to obtain a first SNP set, where the first SNP set includes N SNPs, and N is a positive integer. The processing unit is configured to determine a second SNP set from the first SNP set based on first information of the SNP, where the first information includes at least one of linkage disequilibrium information or a variance, the second SNP set includes M SNPs, M is a positive integer, and M is less than N. The processing unit is further configured to determine a third SNP set from the second SNP set based on a target feature extraction method and a non-linear feature extraction method, where the target feature extraction method includes at least one of a filter feature extraction method, an envelope feature extraction method, or an embedded feature extraction method, the third SNP set includes H SNPs, H is a positive integer, and H is less than M.

In a possible implementation, the first information includes the linkage disequilibrium information, the linkage disequilibrium information includes a linkage disequilibrium LD coefficient, and the processing unit is specifically configured to: delete a target SNP from the first SNP set based on the LD coefficient of the SNP to obtain the second SNP set, where the target SNP is an SNP whose LD coefficient is outside a preset LD coefficient interval.

In a possible implementation, the first information includes the variance, and the processing unit is specifically configured to: when there are a plurality of SNPs with a same variance in the first SNP set, retain one SNP in the plurality of SNPs with the same variance, and delete the other SNPs in the plurality of SNPs to obtain the second SNP set.

In a possible implementation, the processing unit is specifically configured to: determine a fourth SNP set from the second SNP set based on the target feature extraction method, where the fourth SNP set includes K SNPs, K is a positive integer, and K is less than M and greater than H; and determine the third SNP set from the fourth SNP set based on the non-linear feature extraction method.

In a possible implementation, the processing unit is specifically configured to: remove an SNP from the second SNP set based on the target feature extraction method; and when a coefficient of determination of remaining SNPs in the second SNP set is greater than a first threshold or a first remaining ratio is less than a first ratio threshold, determine the remaining SNPs in the second SNP set as the fourth SNP set, where the first remaining ratio is a ratio of a quantity of the remaining SNPs in the second SNP set to M.

In a possible implementation, the processing unit is specifically configured to: remove an SNP from the fourth SNP set based on the non-linear feature extraction method; and when a coefficient of determination of remaining SNPs in the fourth SNP set is greater than a second threshold or a second remaining ratio is less than a second ratio threshold, determine the remaining SNPs in the fourth SNP set as the third SNP set, where the second remaining ratio is a ratio of a quantity of the remaining SNPs in the fourth SNP set to M.

According to a third aspect, an embodiment of this application further provides a data processing apparatus. The data processing apparatus includes at least one processor. When the at least one processor executes program code or instructions, the method in any one of the first aspect or the possible implementations of the first aspect is implemented.

Optionally, the data processing apparatus may further include at least one memory, and the at least one memory is configured to store the program code or the instructions.

According to a fourth aspect, an embodiment of this application further provides a chip, including an input interface, an output interface, and at least one processor. Optionally, the chip further includes a memory. The at least one processor is configured to execute code in the memory. When the at least one processor executes the code, the chip implements the method according to any one of the first aspect or the possible implementations of the first aspect.

Optionally, the chip may be an integrated circuit.

According to a fifth aspect, an embodiment of this application further provides a computer-readable storage medium, configured to store a computer program. The computer program is used to implement the method in the first aspect or any possible implementation of the first aspect.

According to a sixth aspect, an embodiment of this application further provides a computer program product including instructions. When the computer program product runs on a computer, the computer is enabled to implement the method in the first aspect or any possible implementation of the first aspect.

The data processing apparatus, the computer storage medium, the computer program product, and the chip provided in this embodiment are all configured to perform the method provided above. Therefore, for beneficial effect that can be achieved by the data processing apparatus, the computer storage medium, the computer program product, and the chip, refer to the beneficial effect in the method provided above. Details are not described herein again.

## BRIEF DESCRIPTION OF DRAWINGS

To describe technical solutions in embodiments of this application more clearly, the following briefly describes accompanying drawings for describing embodiments. It is clear that the accompanying drawings in the following descriptions show merely a part of embodiments of this application, and a person of ordinary skill in the art may still derive other accompanying drawings from these accompanying drawings without creative efforts.

FIG. 1 is a diagram of a structure of a data processing system according to an embodiment of this application;
FIG. 2 is a schematic flowchart of a data processing method according to an embodiment of this application;
FIG. 3 is a diagram of a structure of a data processing apparatus according to an embodiment of this application;
FIG. 4 is a diagram of a structure of a chip according to an embodiment of this application;
FIG. 5 is a diagram of a structure of an electronic device according to an embodiment of this application; and
FIG. 6 is a diagram of a structure of still another data processing apparatus according to an embodiment of this application.

## DESCRIPTION OF EMBODIMENTS

The following clearly and completely describes technical solutions of embodiments of this application with reference to accompanying drawings in embodiments of this application. It is clear that the described embodiments are merely a part but not all of embodiments of this application. All other embodiments obtained by a person of ordinary skill in the art based on embodiments of this application without creative efforts shall fall within the protection scope of embodiments of this application.

The term "and/or" in this specification describes only an association relationship for describing associated objects and represents that three relationships may exist. For example, A and/or B may represent the following three cases: Only A exists, both A and B exist, and only B exists.

In this specification and the accompanying drawings of embodiments of this application, the terms "first", "second", and the like are intended to distinguish between different objects or distinguish between different processing of a same object, but do not indicate a particular order of the objects.

In addition, the terms "including", "having", and any other variants thereof mentioned in descriptions of embodiments of this application are intended to cover a non-exclusive inclusion. For example, a process, a method, a system, a product, or a device that includes a series of steps or units is not limited to the listed steps or units, but optionally further includes another unlisted step or unit, or optionally further includes another inherent step or unit of the process, the method, the product, or the device.

It should be noted that, in descriptions of embodiments of this application, terms such as "example" or "for example" are used to represent giving an example, an illustration, or a description. Any embodiment or design solution described as "example" or "for example" in embodiments of this application should not be construed as being more preferential or advantageous than other embodiments or design solutions. Exactly, the word "example", "for example", or the like is used to present a related concept in a specific manner.

First, terms used in embodiments of this application are explained and described.

High-dimensional low-sample data is data with high data dimensionality, and a small absolute quantity of samples, or a quantity of samples far less than a data dimensionality feature.

Linkage disequilibrium is a probability that both alleles belonging to two or more genetic loci appear on a same chromosome, and is higher than a frequency of random occurrence. Simply put, as long as two genes are not inherited completely independently, linkage is shown to some extent. This is referred to as "linkage disequilibrium". The linkage disequilibrium may occur in different regions on a same chromosome, or may occur on different chromosomes.

The alleles are usually "a pair of genes, located in same positions on a pair of homologous chromosomes, that control relative traits". An allele may appear in one of two or more genes on a specific locus of a chromosome. If a gene on one locus exists in more than two states, the gene is referred to as "multiple alleles". If two genes in a pair of alleles are identical, an individual is a homozygote for that trait. If the two alleles are different, the individual is a heterozyme for that trait. During heterozygous pairing, a dominant allele makes a trait of a recessive allele unexpressed.

For linkage disequilibrium (linkage disequilibrium, LD) decay, generally, closer two SNPs (SNP loci) in a genome indicates a stronger correlation and a larger LD coefficient. Otherwise, an LD coefficient is smaller. In other words, as a distance between the loci increases, the LD coefficient generally decreases slowly. This rule is usually presented in an LD decay diagram. A decay speed of the LD coefficient varies with different subpopulations. The LD decay speed varies greatly with different populations of a same species due to different genetic backgrounds of the different populations. Domestication selection may lead to a decrease in genetic diversity of a population, and an increase in a correlation between loci (linkage degrees). Therefore, a population with a higher degree of domestication and a larger selection intensity generally has a slowest LD decay speed. For example, cultivated rice usually has a larger LD decay distance than wild rice. Similarly, a decrease in genetic diversity of the population caused by natural selection and genetic drift also decreases the LD decay speed.

A D value, a basic unit of LD, is a measurement of a deviation between an observed haplotype frequency and an expected frequency in an equilibrium state. Although D can express a basic meaning of LD well, due to strict dependence on an allele frequency (allele frequency), D is not suitable to express an actual LD strength, especially in mutual comparison between LD values in different studies. D' and $r^2$ are most important in symbols commonly used to measure LD. Both are based on D and have respective characteristics and usages.

Filter feature extraction method means to first perform feature selection on a dataset and then train a learner. A feature selection process is independent of the subsequent learner. It is equivalent that "filtering" is first performed on initial features, and then features obtained through filtering are used to train a model. The filter feature extraction method includes a filter feature extraction method for removing a low variance, a filter feature extraction method for single-variable feature selection, and another filter feature extraction method.

In the filter feature extraction method for removing the low variance, it is assumed that feature values of a specific feature are only 0 and 1, and if feature values of 95% of instances in all input samples are 1, it can be considered that the feature has little effect; or if 100% of the instances in all the input samples are 1, this feature is meaningless. This method can be used only when the feature values are discrete variables. If the feature values are continuous variables, this method can be used after the continuous variables are discretized. In addition, in practice, it is generally uncommon to have a feature for which more than 95% of values are the same. Therefore, this method is simple but is impractical. This method can be used as pre-processing of feature selection. First, a feature whose value changes slightly is removed, and then an appropriate feature selection method is selected from the feature selection methods mentioned below for further feature selection.

In the filter feature extraction method for single-variable feature selection (single-variable feature selection), a principle of the single-variable feature selection is to separately calculate a statistical indicator of each variable, determine which indicator is important based on the indicator, and remove an unimportant indicator. Specifically, a chi-squared test may be used for a classification problem of the single-variable feature selection. A Pearson correlation coefficient can be used for a regression problem of the single-variable feature selection.

The Pearson correlation coefficient is a simplest method that can help understand a relationship between a feature and a response variable. The method measures a linear correlation between variables. A value range of a result is [-1, 1], where -1 indicates a complete negative correlation, +1 indicates a complete positive correlation, and 0 indicates no linear correlation.

An envelope feature extraction method means to continuously select a feature subset from an initial feature set, train a learner, and evaluate the subset based on performance of the learner until an optimal subset is selected. In the envelope feature extraction method, an exhaustive method is mostly easy to think to search the feature subset. In addition, a random strategy can be used for searching the subset in a Las Vegas method framework (Las Vegas wrapper, LVW). However, in the LVW algorithm, the random strategy is used for searching the feature subset. Therefore, each time the feature subset is evaluated, the learner needs to be trained, resulting in high computing overheads. If there are a large quantity of initial features, the algorithm may run for a long time without meeting a stop condition. If the running time is limited, the algorithm may fail to produce a solution. Therefore, a greedy algorithm may also be used, for example, forward search (a feature is gradually added to an optimal subset until model performance cannot be improved by adding the feature), backward search, and bidirectional search (combining forward search and backward search).

For an embedded feature extraction method, in the filter feature selection method and the envelope feature selection method, the feature selection process is obviously different from the learner training process. However, for embedded feature selection, feature selection is automatically performed in a learner training process. Most commonly used embedded feature extraction methods are L1 regularization and L2 regularization. A larger regularization term indicates a simpler model and a smaller coefficient. When the regularization term increases to a specific extent, all feature coefficients tend to 0. In this process, a part of feature coefficients first change to 0. This implements the feature selection process. Logistic regression, linear regression, and a decision tree can serve as base learners for regularized feature selection. Only an algorithm that can obtain a feature coefficient or feature importance can be used as the base learner for embedded selection.

A Hilbert-Schmidt independence criterion (Hilbert-Schmidt Independence Criterion, HSIC) regression model (Lasso) feature extraction method is a kernel-based feature selection algorithm. An important feature is selected to explain prediction of HSIC Lasso based on weights of different features in HSIC Lasso. A format of HSIC Lasso is as follows:

$$\max_{\alpha \geq 0} \sum_{k=1}^{d} \alpha_k \text{HSIC}(f_k, y) - \frac{1}{2} \sum_{k,k'=1}^{d} \alpha_k \alpha_{k'} \text{HSIC}(f_k, f_{k'}) - \lambda ||\alpha||_1$$

$\alpha = [\alpha_1, \alpha_2, \dots, \alpha_d]^T$ is a feature weight, $\lambda$ is a regular parameter, $f_k = \left[x_1^{(k)}, x_2^{(k)}, \dots, x_n^{(k)}\right]^T$ is a $k^{th}$ feature value in data, and y is a real value.

Ridge regression (Ridge regression) is a biased estimation regression method dedicated to collinear data analysis, and is essentially an improved least squares estimation method. Unbiasedness of the least squares method is abandoned, so that ridge regression is a more practical and reliable regression method in which a regression coefficient is obtained at costs of losing some information and reducing accuracy, and provides better fitting for ill-conditioned data than the least squares method.

Lasso regression (Lasso regression) is characterized in constructing a generalized linear model. The generalized linear model includes a one-dimensional continuous dependent variable, a multi-dimensional continuous dependent variable, a non-negative times dependent variable, a binary discrete dependent variable, and a multi-variate discrete dependent variable. In addition, Lasso can process all dependent variables regardless of whether the dependent variables are continuous or discrete. In general, Lasso has an extremely low requirement on data and therefore is widely used. In addition, Lasso can perform variable selection and reduce complexity of the model. Variable selection herein is that not all variables are put into the model for fitting, but variables are selectively put into the model to obtain a better performance parameter. Complexity adjustment is to control complexity of the model by using a series of parameters to avoid overfitting. For a linear model, complexity is directly related to a quantity of variables in the model. More variables indicate higher complexity of the model. More variables can usually provide a seemingly better model during fitting, but also face a risk of overfitting.

Elastic net regularization (Elastic net regularization) is a method that combines L1 regularization and L2 regularization. Elastic net regularization can control complexity and sparseness of a model and avoid instability that may be produced in L1 regularization in some cases.

L1 regularization is a widely used regularization method, and introduces an L1 norm of a model parameter in a loss function to penalize a parameter size. This regularization method tends to set a part of parameters to zero, and therefore can be used for feature selection, making the model more sparse.

L2 regularization is another common regularization method, and introduces a L2 norm of a model parameter in a loss function to penalize a parameter size. Different from L1 regularization, L2 regularization does not tend to set a parameter to zero, but controls complexity of the model by reducing the parameter size.

Support vector regression (support vector regression, SVR) is a supervised learning algorithm used to guess a reunion value. Support vector regression uses a similar principle as that of a support vector machine (support vector machine, SVM). A basic underlying idea of SVR is to find a best fitting line. In SVR, the best fitting line is a super-stereo with a largest quantity of points.

A lightweight gradient boosting machine (light gradient boosting machine, light GBM) is a boosting (Boosting) method. Boosting is a process of using a series of linear model combinations to complete a model task. In Boosting learning, each sub-model, gradually determined between models, is superimposed into a composite model. In this process, it is ensured that a loss function gradually decreases as the sub-model increases.

A random forest (Random Forest) is an algorithm that ensembles a plurality of trees based on an idea of ensemble learning. A basic unit of the random forest is a decision tree, and an essence of the random forest is a branch of machine learning, that is, an ensemble learning (Ensemble Learning) method. A name of the random forest includes two keywords: "random" and "forest". "Forest" is easy to understand. One is referred to as a tree, and hundreds or thousands of trees can be referred to as a forest. Such a metaphor is very appropriate. Actually, this is also a reflection of a main idea of the random forest, that is, an ensemble idea. From an intuitive perspective for explanation, each decision tree is a classifier (it is assumed that the classification problem is discussed). Therefore, for an input sample, N trees have N classification results. The random forest ensembles all classification voting results and specifies a category with most votes as a final output.

A Pearson correlation coefficient (Pearson correlation coefficient, PCC) is a method to measure a correlation between two variables. The Pearson correlation coefficient is a value between 1 and -1, where 1 indicates a complete positive correlation between variables, 0 indicates no linear correlation, and -1 indicates a complete negative correlation. A formula for calculating the PCC is as follows:

$$\rho = \frac{\sum_{i=1}^{N}(x_i - \bar{x})(y_i - \bar{y})}{\sqrt{\sum_{i=1}^{N}(x_i - \bar{x})^2 \sum_{i=1}^{N}(y_i - \bar{y})^2}}$$

$\rho$ is the PPC, $\bar{x}$ is a mean of a variable x, $\bar{y}$ is a mean of a variable y, and a value range of the PCC is [-1, 1]. There is no correlation when the PCC is close to 0. When the PCC is closer to 1 (-1), it indicates that there is a stronger positive correlation (negative correlation) between two variables.

A Spearman correlation coefficient (Spearman correlation coefficient, SCC) is used to measure a correlation between two variables. The Spearman correlation coefficient indicates a correlation direction of X (independent variable) and Y (dependent variable). If Y tends to increase as X increases, the Spearman correlation coefficient is positive. If Y tends to decrease as X increases, the Spearman correlation coefficient is negative. A value range of the Spearman correlation coefficient is -1 to 1, where -1 indicates a complete negative correlation, 0 indicates no correlation, and 1 indicates a complete positive correlation. A formula for calculating the SCC is a change form of the formula for calculating the PCC. The formula for calculating the SCC is as follows:

$$\rho_s = \frac{\sum_{i=1}^{N}(R_i - \bar{R})(S_i - \bar{S})}{\sqrt{\sum_{i=1}^{N}(R_i - \bar{R})^2 \sum_{i=1}^{N}(S_i - \bar{S})^2}}$$

$\rho_s$ is the SCC, and $R_i$ and $S_i$ are respectively levels of observed values $x_i$ and $y_i$, that is, values of the SCC are sorted in ascending order. The SCC evaluates a monotonic relationship between the two variables and determines whether a trend of together change is reflected between the variables.

A mean absolute error (mean absolute error, MAE) reflects an absolute cumulative deviation between predicted values and true values of a plurality of samples. The mean absolute error is a mean of absolute values of deviations between all single observed values and an arithmetic mean. The mean absolute error can avoid a problem that errors are mutually canceled, and therefore can accurately reflect an actual prediction error. A formula for calculating the MAE is as follows:

$$MAE = \frac{1}{n}\sum_{i=1}^{n}|y_i - \hat{y}_i|$$

n is a quantity of samples, $y_i$ is an actual observed value, and $\hat{y}_i$ is a real value.

A mean squared error (mean squared error, MSE) is a measurement that reflects a difference degree between an estimating amount and an estimated amount. A formula for calculating the MSE is as follows:

$$MSE = \frac{1}{n}\sum_{i=1}^{n}(y_i - \hat{y}_i)^2$$

A root mean squared error (root mean squared error, RMSE) is a root mean square of prediction errors, and is used to measure accuracy of regression prediction. A formula for calculating the RMSE is as follows:

$$RMSE = \sqrt{\frac{1}{n}\sum_{i=1}^{n}(y_i - \hat{y}_i)^2}$$

$R^2$, namely, a coefficient of determination (coefficient of determination), is a statistical indicator that quantifies to represent a correlation between qualitative variables or factors, and is commonly used to measure a strength of a linear relationship between two variables. The coefficient of determination is a quantitative measurement indicator whose value range is from 0 to 1 and can also be considered as a ratio reflecting a total quantity of variations of a variable. If the ratio is greater than 0.8, the linear relationship between the two variables is strong. If the value is less than 0.4, there is no linear relationship between the two variables. The coefficient of determination is one of most important measurement indicators in statistical analysis of correlation analysis. A formula for calculating the coefficient of determination is as follows:

$$R^2 = PCC^2$$

A coefficient of variation (coefficient of variation, CV) is a statistical measurement that measures a variation degree of each observed value in data. When variation degrees of two or more pieces of data are compared, if a measurement unit is the same as a mean, a standard deviation may be directly used for comparison. If the unit and (or) the mean are (is) different, the standard deviation cannot be used to compare the variation degrees of the two or more pieces of data. Instead, a ratio (relative value) of the standard deviation to the mean is used. A formula for calculating the mean CV of the RMSE is as follows:

$$CV = \frac{RMSE}{Mean\ Value}$$

CV is the mean CV of the RMSE, and Mean Value is a mean of the RMSE.

An SNP is mainly a DNA sequence polymorphism caused by a variation of a single nucleotide at a genome level. The SNP, as one of most common biological genetic variations, accounts for more than 90% of all known polymorphisms. The SNP is widely present in biological genomes, occurring on average once every 300 base pairs, with an estimated total quantity of 3 million or even more. The SNP is a biallelic marker, and is caused by transition or transversion of a single base, or insertion or absence of a base. The SNP may be in a gene sequence, or may be in a non-coding sequence outside a gene. During gene selection, a core SNP with high heritability needs to be selected from biological SNPs.

However, only a few of tens of thousands of biological SNPs are core SNPs with a high heritability characteristic.

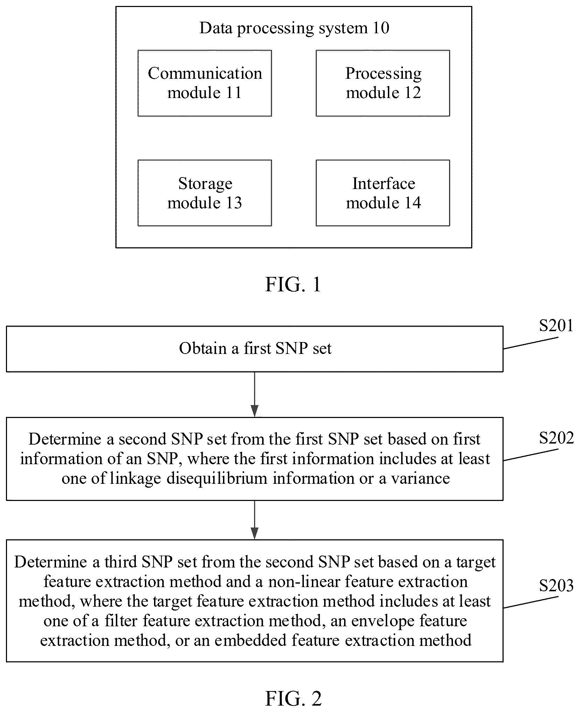

Therefore, an embodiment of this application provides a data processing method, and the method may be applied to a data processing system. FIG. 1 is a functional block diagram of a data processing system 10 according to an embodiment of this application. As shown in FIG. 1, the data processing system 10 may include various submodules, for example, a communication module 11, a processing module 12, a storage module 13, and an interface module 14.

Optionally, the data processing system 10 may include more or fewer submodules, and each submodule may include a plurality of components. In addition, each submodule and component of the data processing system 10 may be interconnected in a wired or wireless manner.

The communication module 11 may include a wireless communication system, and the wireless communication system may communicate with one or more devices in wireless manner directly or by using a communication network. For example, the wireless communication system may use 3rd generation mobile communication technology (3rd generation mobile communication technology, 3G) cellular communication, for example, code division multiple access (code division multiple access, CDMA), a global system for mobile communications (global system for mobile communications, GSM), or a general packet radio service (general packet radio service, GPRS), or 4th generation mobile communication technology (4th generation mobile communication technology, 4G) cellular communication, for example, long term evolution (long term evolution, LTE), or 5th generation mobile communication technology (the 5th generation mobile communication technology, 5G) cellular communication. The wireless communication system may communicate with a wireless local area network (wireless local area network, WLAN) through wireless fidelity (wireless fidelity, Wi-Fi). In some embodiments, the wireless communications system may directly communicate with a device through an infrared link, Bluetooth, or ZigBee (ZigBee).

The processing module 12 may be any conventional processor, for example, a commercially available CPU. Alternatively, the processing module 12 may further include a graphics processing unit (Graphics Processing Unit, GPU), a field programmable gate array (Field Programmable Gate Array, FPGA), a system on chip (System on Chip, SOC), an application-specific integrated circuit (Application-specific Integrated Circuit, ASIC), or a combination thereof.

The storage module 13 is configured to store instructions. The instructions may be computer programs.

The storage module 13 may be a volatile memory or a non-volatile memory, or may include both a volatile memory and a non-volatile memory. The non-volatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM), used as an external cache. Through example but not limitative description, many forms of RAMs may be used, for example, a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM). The storage module 13 may alternatively be a compact disc read-only memory (compact disc read-only memory, CD-ROM) or another optical disc storage, an optical disc storage (including a compact disc, a laser disc, an optical disc, a digital versatile disc, a Blu-ray disc, or the like), a magnetic disk storage medium, another magnetic storage device, or the like. It should be noted that the memory of the systems and methods described in this specification includes but is not limited to these and any memory of another proper type.

The interface module 14 may be used to send or receive data, instructions, or information. The processing module 12 may process the data, the instructions, or other information received through the interface module 14, and may send processed information through the interface module 14.

Optionally, the foregoing components are merely examples. In actual application, components in the foregoing modules may be added or deleted based on an actual requirement. FIG. 1 should not be understood as any limitation on embodiments of the present invention.

FIG. 2 shows a data processing method according to an embodiment of this application. The method includes the following steps.

S201: Obtain a first SNP set.

The first SNP set includes N SNPs, and N is a positive integer.

For example, N soybean SNPs may be obtained, and a set including the obtained N soybean SNPs is used as the first SNP set.

In a possible implementation, the N SNPs included in the first SNP set may be high-dimensional low-sample data.

S202: Determine a second SNP set from the first SNP set based on first information of the SNP.

The first information includes at least one of linkage disequilibrium information or a variance, the second SNP set includes M SNPs, M is a positive integer, and M is less than N. The linkage disequilibrium information may include a linkage disequilibrium (linkage disequilibrium, LD) coefficient.

In a possible implementation, when there are a plurality of SNPs with a same variance in the first SNP set, one SNP in the plurality of SNPs with the same variance may be retained, and the other SNPs in the plurality of SNPs may be deleted to obtain the second SNP set.

For example, when the first SNP set includes 1000 SNPs whose variances are all 0.45, a 1st SNP that appears in the 1000 SNPs whose variances are 0.45 may be retained in the first SNP set, and remaining 999 SNPs whose variances are 0.45 are deleted from the first SNP set.

It may be understood that the SNPs with the same variance may also have a same genetic characteristic. Therefore, only one SNP in the plurality of SNPs with the same variance may be retained, and the other SNPs in the plurality of SNPs with the same variance are deleted from obtained SNP data, to perform data dimension reduction on the obtained SNP data; and then feature selection is performed on the dimension-reduced data by using a target feature extraction method and a non-linear feature extraction method, to select a core SNP with a high heritability characteristic from biological SNPs. In addition, in this embodiment of this application, an SNP that is strongly correlated with a dependent variable (phenotype) is also selected from the plurality of obtained SNPs based on the variance and the linkage disequilibrium information of the SNP.

In a possible implementation, a target SNP may be deleted from the first SNP set based on an LD coefficient of the SNP to obtain the second SNP set, where the target SNP is an SNP whose LD coefficient is outside a preset LD coefficient interval.

For example, the LD coefficient of the SNP may be determined based on a corresponding frequency of a feature of the SNP in a population, and the target SNP is deleted from the first SNP set based on the LD coefficient of the SNP, to obtain the second SNP set.

For example, it is assumed that there are two SNPs $(x_i, x_j)$. Bases of the two SNPs are represented as (A, B), and alleles are respectively A, a, B, b, corresponding to frequencies $\pi_A$, $\pi_a$, $\pi_B$, $\pi_b$ in the population. The two SNPs have four haplotypes AB, Ab, aB, ab, corresponding to frequencies $\pi_{AB}$, $\pi_{Ab}$, $\pi_{aB}$, $\pi_{ab}$. $D_{ab} = \pi_{AB} - \pi_A \pi_B$ and an LD coefficient are calculated. Then, the SNP whose LD coefficient is outside the preset LD coefficient interval is deleted from the first SNP set based on an LD coefficient of each SNP obtained through calculation.

The LD coefficient satisfies: when $D_{ab} > 0, |D'| = \frac{(D_{ab})^2}{\min (\pi_A \pi_B, \pi_a \pi_b)}$ ; when $D_{ab} < 0, |D'| = \frac{(D_{ab})^2}{\min (\pi_A \pi_b, \pi_a \pi_B)}$ ; and $r^2 = \frac{(D_{ab})^2}{\pi_A \pi_b \pi_a \pi_B}$, where D' = 0, $r^2$ = 0 is in a complete linkage equilibrium state, and D' = 1, $r^2$ = 1 is in a complete linkage disequilibrium state. A higher measurement from 0 to 1 indicates a higher LD and stronger linkage.

S203: Determine a third SNP set from the second SNP set based on the target feature extraction method and the non-linear feature extraction method.

The target feature extraction method includes at least one of a filter feature extraction method, an envelope feature extraction method, or an embedded feature extraction method, the third SNP set includes H SNPs, H is a positive integer, and H is less than M.

In a possible implementation, a fourth SNP set may be determined from the second SNP set based on the target feature extraction method. The third SNP set is determined from the fourth SNP set based on the non-linear feature extraction method. The fourth SNP set includes K SNPs, K is a positive integer, and K is less than M and greater than H.

In a possible implementation, an SNP is removed from the second SNP set based on the target feature extraction method; and when a coefficient of determination of remaining SNPs in the second SNP set is greater than a first threshold or a first remaining ratio is less than a first ratio threshold, the remaining SNPs in the second SNP set are determined as the fourth SNP set, where the first remaining ratio is a ratio of a quantity of the remaining SNPs in the second SNP set to M.

For example, the SNP may be selected and removed from the second SNP set based on the filter feature extraction method, the envelope feature extraction method, and the embedded feature extraction method. When the coefficient of determination of the remaining SNPs in the second SNP set is greater than 0.7 or the first remaining ratio is less than 20% (in other words, 80% SNPs have been selected and removed from the second SNP set), the remaining SNPs in the second SNP set are determined as the fourth SNP set.

Correspondingly, when the coefficient of determination of the remaining SNPs is less than the first threshold and the first remaining ratio is greater than the first ratio threshold, the SNP may continue to be removed from the second SNP set based on the target feature extraction method until the coefficient of determination of the remaining SNPs in the second SNP set is greater than the first threshold or the first remaining ratio is less than the first ratio threshold.

It can be learned that, according to the method provided in this embodiment of this application, a representative SNP in each region is determined from the plurality of obtained SNPs based on the variance and the linkage disequilibrium information of the SNP, and the remaining redundant SNPs are removed. Therefore, data dimension reduction is performed on the obtained SNP data, then selection is further performed on the plurality of obtained SNPs by using the target feature extraction method based on the coefficient of determination and a quantity of the remaining SNPs, and feature selection is performed, by using a non-linear feature, on the SNPs obtained through further selection, to select the core SNP with the high heritability characteristic from the biological SNPs. In addition, selection is further performed on the plurality of obtained SNPs by using the target feature extraction method based on the coefficient of determination and the quantity of the remaining SNPs, so that an SNP of a penalty term of a correlation between features can be obtained.

In a possible implementation, a PCC of the remaining SNPs may be determined, and the coefficient of determination of the SNP may be determined based on the PCC of the remaining SNPs.

In a possible implementation, an SNP may be removed from the fourth SNP set based on the non-linear feature extraction method. When a coefficient of determination of remaining SNPs in the fourth SNP set is greater than a second threshold or a second remaining ratio is less than a second ratio threshold, the remaining SNPs in the fourth SNP set are determined as the third SNP set, where the second remaining ratio is a ratio of a quantity of the remaining SNPs in the fourth SNP set to M.

For example, the SNP may be selected and removed from the fourth SNP set by using the non-linear feature extraction method. When the coefficient of determination of the remaining SNPs in the fourth SNP set is greater than 0.75 or the second remaining ratio is less than 1% (that is, 99% SNPs have been selected and removed from the second SNP set), the remaining SNPs in the fourth SNP set are determined as the third SNP set.

Correspondingly, when the coefficient of determination of the remaining SNPs is less than the second threshold and the second remaining ratio is greater than the second ratio threshold, the SNP may continue to be removed from the fourth SNP set based on the target feature extraction method until the coefficient of determination of the remaining SNPs in the fourth SNP set is greater than the second threshold or the second remaining ratio is less than the second ratio threshold.

It can be learned that, according to the method provided in this embodiment of this application, the representative SNP in each region is determined from the plurality of obtained SNPs based on the variance and the linkage disequilibrium information of the SNP, and the remaining redundant SNPs are removed. Therefore, data dimension reduction is performed on the obtained SNP data, then selection is further performed on the plurality of obtained SNPs by using the target feature extraction method, and selection is performed again, from the plurality of obtained SNPs based on the coefficient of determination and a quantity of the remaining SNPs by using a non-linear feature, on the SNPs obtained through further selection, to select the core SNP with the high heritability characteristic from the biological SNPs.

Optionally, the non-linear feature extraction method includes but is not limited to an HSIC Lasso feature extraction method.

A specific implementation method for determining the coefficient of determination of the SNP is not limited in this embodiment of this application, and may be any method that can be thought of by a person skilled in the art. For example, the coefficient of determination of the SNP may be determined according to a regression integration algorithm (Ridge regression, Lasso regression, Elastic net regularization, SVR, or LightGBM).

It should be noted that one SNP in a plurality of SNPs in one region may represent types of most SNPs in the region. In this embodiment of this application, the representative SNP in each region is determined from the plurality of obtained SNPs based on the variance and the linkage disequilibrium information of the SNP, and the other redundant SNPs are removed. Therefore, data dimension reduction is performed on the obtained SNP data, and then feature selection is performed on the dimension-reduced data by using the target feature extraction method and the non-linear feature extraction method, to select the core SNP with the high heritability characteristic from the biological SNPs. In addition, in this embodiment of this application, an SNP that is strongly correlated with a dependent variable (phenotype) is also selected from the plurality of obtained SNPs based on the variance and the linkage disequilibrium information of the SNP.

Table 1 shows a prediction result obtained by predicting a soybean phenotype in the data processing method provided in this embodiment of this application. It can be learned from Table 1 that a prediction correlation coefficient PCC indicator obtained by using the data processing method provided in this embodiment of this application reaches more than 94%, and is close to heritability of 95%. A quantity of feature SNPs obtained through selection by jointing preprocessing and HSIC LASSO is 440, and is reduced by 99.7%. An overall result is improved. Compared with a test result in a related technology, the data processing method provided in this embodiment of this application is advanced in predicting a phenotype based on genotype data.

Table 1

| | Ridge regression best linear unbiased prediction | Ridge regression | Lasso regression | Elastic net regularization | Support vector regression | Light gradient boosting machine | Random forest | Optimized support vector regression |
|---|---|---|---|---|---|---|---|---|
| Spearman correlation coefficient | 0.820685 | 0.932586 | 0.923029 | 0.932946 | 0.933159 | 0.922480 | 0.918715 | 0.930643 |
| Pearson correlation coefficient | 0.823269 | 0.939760 | 0.932528 | 0.939450 | 0.938405 | 0.936616 | 0.931155 | 0.943494 |
| Mean absolute error | 2.938232 | 1.523224 | 1.648004 | 1.525202 | 1.507681 | 1.521075 | 1.680071 | 1.440345 |
| Mean squared error | 15.687203 | 4.775989 | 5.277461 | 4.814200 | 4.876605 | 4.999318 | 5.792596 | 4.460974 |

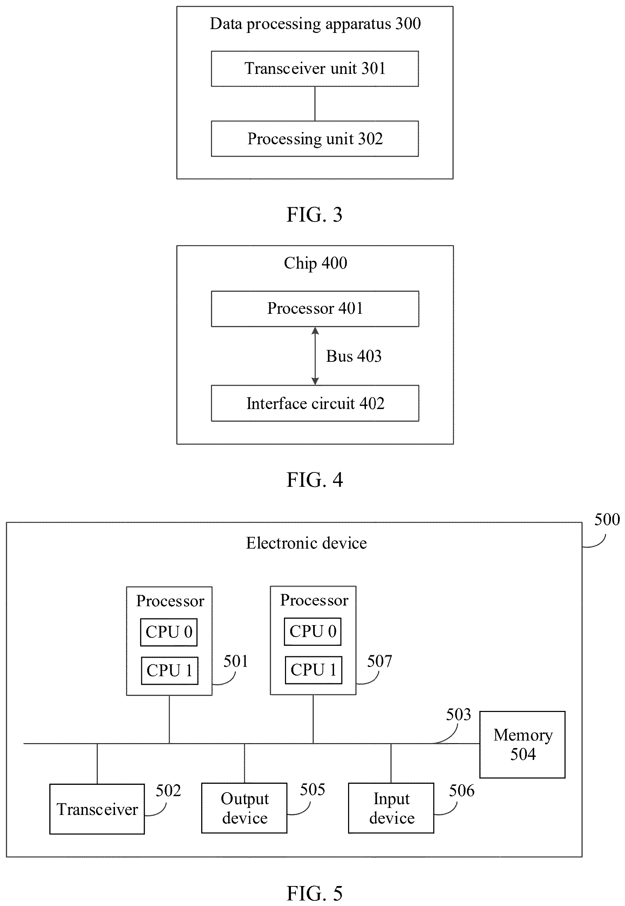

The following describes, with reference to FIG. 3, a data processing apparatus configured to perform the data processing method.

It may be understood that, to implement the foregoing functions, the data processing apparatus includes a corresponding hardware and/or software module for performing the functions. With reference to the example algorithm steps described in embodiments disclosed in this specification, embodiments of this application can be implemented in a form of hardware or a combination of hardware and computer software. Whether a function is performed by hardware or hardware driven by computer software depends on particular applications and design constraints of the technical solutions. A person skilled in the art may use different methods to implement the described functions for each particular application with reference to embodiments, but it should not be considered that the implementation goes beyond the scope of embodiments of this application.

In embodiments of this application, a data processing apparatus may be divided into functional modules based on the foregoing method examples. For example, each functional module may be obtained through division based on each corresponding function, or two or more functions may be integrated into one processing module. The integrated module may be implemented in a form of hardware. It should be noted that module division in embodiments is an example and is merely logical function division. In practice, there may be another division manner.

When functional modules are obtained through division based on corresponding functions, FIG. 3 is a diagram of a possible composition of the data processing apparatus in the foregoing embodiments. As shown in FIG. 3, a data processing apparatus 300 may include a transceiver unit 301 and a processing unit 302.

The transceiver unit 301 is configured to obtain a first SNP set, where the first SNP set includes N SNPs, and N is a positive integer.

The processing unit 302 is configured to determine a second SNP set from the first SNP set based on first information of the SNP, where the first information includes at least one of linkage disequilibrium information or a variance, the second SNP set includes M SNPs, M is a positive integer, and M is less than N.

The processing unit 302 is further configured to determine a third SNP set from the second SNP set based on a target feature extraction method and a non-linear feature extraction method, where the target feature extraction method includes at least one of a filter feature extraction method, an envelope feature extraction method, or an embedded feature extraction method, the third SNP set includes H SNPs, H is a positive integer, and H is less than M.

In a possible implementation, the first information includes the linkage disequilibrium information, the linkage disequilibrium information includes a linkage disequilibrium LD coefficient, and the processing unit is specifically configured to: delete a target SNP from the first SNP set based on the LD coefficient of the SNP to obtain the second SNP set, where the target SNP is an SNP whose LD coefficient is outside a preset LD coefficient interval.

In a possible implementation, the first information includes the variance, and the processing unit 302 is specifically configured to: when there are a plurality of SNPs with a same variance in the first SNP set, retain one SNP in the plurality of SNPs with the same variance, and delete the other SNPs in the plurality of SNPs to obtain the second SNP set.

In a possible implementation, the processing unit 302 is specifically configured to: determine a fourth SNP set from the second SNP set based on the target feature extraction method, where the fourth SNP set includes K SNPs, K is a positive integer, and K is less than M and greater than H; and determine the third SNP set from the fourth SNP set based on the non-linear feature extraction method.

In a possible implementation, the processing unit 302 is specifically configured to: remove an SNP from the second SNP set based on the target feature extraction method; and when a coefficient of determination of remaining SNPs in the second SNP set is greater than a first threshold or a first remaining ratio is less than a first ratio threshold, determine the remaining SNPs in the second SNP set as the fourth SNP set, where the first remaining ratio is a ratio of a quantity of the remaining SNPs in the second SNP set to M.

In a possible implementation, the processing unit 302 is specifically configured to: remove an SNP from the fourth SNP set based on the non-linear feature extraction method; and when a coefficient of determination of remaining SNPs in the fourth SNP set is greater than a second threshold or a second remaining ratio is less than a second ratio threshold, determine the remaining SNPs in the fourth SNP set as the third SNP set, where the second remaining ratio is a ratio of a quantity of the remaining SNPs in the fourth SNP set to M.

An embodiment of this application further provides a chip. FIG. 4 is a diagram of a structure of a chip 1000. The chip 400 includes one or more processors 401 and an interface circuit 402. Optionally, the chip 400 may further include a bus 403.

The processor 401 may be an integrated circuit chip and has a signal processing capability. In an implementation process, steps of the foregoing data processing method can be implemented by using a hardware integrated logic circuit in the processor 401, or by using an instruction in a form of software.

Optionally, the processor 401 may be a general-purpose processor, a digital signal processor (digital signal processor, DSP), an application-specific integrated circuit (application specific integrated circuit, ASIC), a field-programmable gate array (field-programmable gate array, FPGA) or another programmable logic device, a discrete gate or a transistor logic device, or a discrete hardware component. The processor 401 may implement or perform the methods and steps that are disclosed in embodiments of this application. The general-purpose processor may be a microprocessor, or the processor may be any conventional processor or the like.

The interface circuit 402 may be used to send or receive data, instructions, or information. The processor 401 may process the data, the instructions, or other information received through the interface circuit 402, and may send processed information through the interface circuit 402.

Optionally, the chip further includes a memory. The memory may include a read-only memory and a random access memory, and provide operation instructions and data for the processor. A part of the memory may further include a non-volatile random access memory (non-volatile random access memory, NVRAM).

Optionally, the memory stores an executable software module or a data structure, and the processor may perform a corresponding operation by invoking the operation instructions stored in the memory (the operation instructions may be stored in an operating system).

Optionally, the chip may be used in the data processing apparatus in embodiments of this application. Optionally, the interface circuit 402 may be configured to output an execution result of the processor 401. For the data processing method provided in one or more embodiments of this application, refer to the foregoing embodiments. Details are not described herein again.

It should be noted that functions corresponding to each of the processor 401 and the interface circuit 402 may be implemented by using a hardware design, or may be implemented by using a software design, or may be implemented by using a combination of software and hardware. This is not limited herein.

FIG. 5 is a diagram of a structure of an electronic device according to an embodiment of this application. An electronic device 500 may be a processor, or a chip or a functional module in a processor. As shown in FIG. 5, the electronic device 500 includes a processor 501, a transceiver 502, and a communication line 503.

The processor 501 is configured to perform any step in the data processing method provided in embodiments of this application, and in a process of performing any step in the data processing method provided in embodiments of this application, may select to invoke the transceiver 502 and the communication line 503 to complete a corresponding operation.

Further, the electronic device 500 may further include a memory 504. The processor 501, the memory 504, and the transceiver 502 may be connected through the communication line 503.

The processor 501 is a processor, a general-purpose processor, a network processor (network processor, NP), a digital signal processor (digital signal processor, DSP), a microprocessor, a microcontroller, a programmable logic device (programmable logic device, PLD), or any combination thereof. The processor 501 may alternatively be another apparatus having a processing function, for example, a circuit, a device, or a software module. This is not limited.

The transceiver 502 is configured to communicate with another device or another communication network. The another communication network may be the Ethernet, a radio access network (radio access network, RAN), a wireless local area network (wireless local area network, WLAN), or the like. The transceiver 502 may be a module, a circuit, a transceiver, or any apparatus that can implement communication.

The transceiver 502 is mainly configured to receive and send commands, information, and the like, and may include a transmitter and a receiver that separately send and receive the commands, the information, and the like. An operation other than receiving and sending the commands, the information, and the like is implemented by the processor.

The communication line 503 is configured to transmit information between components included in the electronic device 500.

In a design, the processor may be considered as a logic circuit, and the transceiver may be considered as an interface circuit.

The memory 504 is configured to store instructions. The instructions may be computer programs.

The memory 504 may be a volatile memory or a non-volatile memory, or may include both a volatile memory and a non-volatile memory. The non-volatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM), used as an external cache. Through example but not limitative description, many forms of RAMs may be used, for example, a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM). The memory 504 may alternatively be a compact disc read-only memory (compact disc read-only memory, CD-ROM) or another optical disc storage, an optical disc storage (including a compact disc, a laser disc, an optical disc, a digital versatile disc, a Blu-ray disc, or the like), a magnetic disk storage medium, another magnetic storage device, or the like. It should be noted that the memory of the systems and methods described in this specification includes but is not limited to these and any memory of another proper type.

It should be noted that the memory 504 may be independent of the processor 501, or may be integrated with the processor 501. The memory 504 may be configured to store instructions, program code, some data, or the like. The memory 504 may be located inside the electronic device 500, or may be located outside the electronic device 500. This is not limited. The processor 501 is configured to execute the instructions stored in the memory 504, to implement the methods provided in the foregoing embodiments of this application.

In an example, the processor 501 may include one or more processors, for example, a processor 0 and a processor 1 in FIG. 5.

In an optional implementation, the electronic device 500 includes a plurality of processors. For example, in addition to the processor 501 in FIG. 5, the electronic device 500 may further include a processor 507.

In an optional implementation, the electronic device 500 further includes an output device 505 and an input device 506. For example, the input device 506 is a device, for example, a keyboard, a mouse, a microphone, or a joystick, and the output device 505 is a device, for example, a display or a speaker (speaker).

It should be noted that the electronic device 500 may be a chip system or a device having a structure similar to that in FIG. 5. The chip system may include a chip, or may include a chip and another discrete device. For actions, terms, and the like in embodiments of this application, refer to each other. This is not limited. In embodiments of this application, names of messages exchanged between devices, names of parameters in the messages, or the like are merely examples. Other names may alternatively be used during specific implementation. This is not limited. In addition, the composition structure shown in FIG. 5 does not constitute a limitation on the electronic device 500. In addition to the components shown in FIG. 5, the electronic device 500 may include more or fewer components than those shown in FIG. 5, or combine some of the components, or have different layouts of the components.

The processor and the transceiver described in this application may be implemented in an integrated circuit (integrated circuit, IC), an analog IC, a radio frequency integrated circuit, a mixed signal IC, an application-specific integrated circuit (application-specific integrated circuit, ASIC), a printed circuit board (printed circuit board, PCB), an electronic device, or the like. The processor and the transceiver may alternatively be manufactured by using various IC process technologies, for example, a complementary metal oxide semiconductor (complementary metal oxide semiconductor, CMOS), an N-channel metal oxide semiconductor (N-channel Metal oxide semiconductor, NMOS), a P-channel metal oxide semiconductor (positive-channel metal oxide semiconductor, PMOS), a bipolar junction transistor (Bipolar Junction Transistor, BJT), a bipolar CMOS (BiCMOS), silicon germanium (SiGe), and gallium arsenide (GaAs).

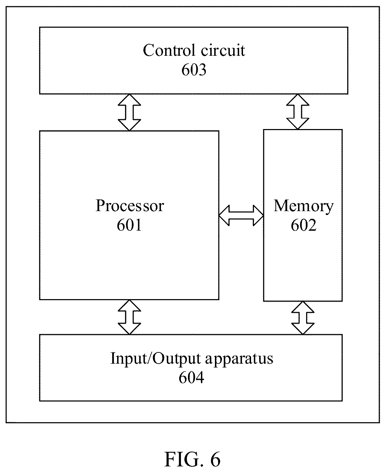

FIG. 6 is a diagram of a structure of a data processing apparatus according to an embodiment of this application. The data processing apparatus may be applicable to scenarios shown in the foregoing method embodiments. For ease of description, FIG. 6 shows only main components of the data processing apparatus, including a processor 601, a memory 602, a control circuit 603, and an input/output apparatus 604. The processor 601 is mainly configured to: process a communication protocol and communication data, execute a software program, and process data of the software program. The memory 602 is mainly configured to store the software program and the data. The control circuit 603 is mainly configured to supply power and transmit various electrical signals. The input/output apparatus 604 is mainly configured to receive data input by a user and output data to the user.

When the data processing apparatus is the processor 601, the control circuit 603 may be a mainboard. The memory 602 includes a medium having a storage function, for example, a hard disk, a RAM, or a ROM. The processor 601 may include a baseband processor 601 and a central processing unit. The baseband processor is mainly configured to process the communication protocol and communication data. The central processing unit is mainly configured to: control the entire data processing apparatus, execute the software program, and process the data of the software program. The input/output apparatus 604 includes a display, a keyboard, a mouse, and the like. The control circuit 603 may further include or be connected to a transceiver circuit or a transceiver, for example, a network cable interface, and is configured to send or receive data or a signal, for example, perform data transmission and communication with another device. Further, the control circuit may further include an antenna, configured to receive and send a radio signal, and configured to perform data/signal transmission with another device.

An embodiment of this application further provides a data processing apparatus. The apparatus includes at least one processor. When the at least one processor executes program code or instructions, the foregoing related method steps are implemented to implement the data processing method in the foregoing embodiments.

Optionally, the apparatus may further include at least one memory, and the at least one memory is configured to store the program code or the instructions.

An embodiment of this application further provides a computer storage medium. The computer storage medium stores computer instructions. When the computer instructions are run on a data processing apparatus, the data processing apparatus is enabled to perform the foregoing related method steps, to implement the data processing method in the foregoing embodiments.

An embodiment of this application further provides a computer program product. When the computer program product runs on a computer, the computer is enabled to perform the foregoing related steps to implement the data processing method in the foregoing embodiments.

An embodiment of this application further provides a data processing apparatus. The apparatus may be specifically a chip, an integrated circuit, a component, or a module. Specifically, the apparatus may include a connected processor and a memory configured to store instructions, or the apparatus includes at least one processor, configured to obtain instructions from an external memory. When the apparatus runs, the processor may execute the instructions, so that the chip performs the data processing method in the foregoing method embodiments.

It should be understood that sequence numbers of the foregoing processes do not mean execution sequences in various embodiments of this application. The execution sequences of the processes should be determined according to functions and internal logic of the processes, and should not be construed as any limitation on the implementation processes of embodiments of this application.

A person of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In the several embodiments provided in this application, it should be understood that the disclosed system, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate components may or may not be physically separate, and parts displayed as components may or may not be physical units, may be located in one location, or may be distributed on a plurality of network units. A part or all of the units may be selected based on actual requirements to achieve the objectives of the solutions of embodiments.

In addition, functional units in embodiments of this application may be integrated into one processing unit, or each of the units may exist alone physically, or two or more units may be integrated into one unit.

When the functions are implemented in a form of software functional unit and sold or used as an independent product, the functions may be stored in a computer-readable storage medium. Based on such an understanding, the technical solutions of this application essentially, or the part contributing to the conventional technology, or some of the technical solutions may be implemented in a form of a software product. The computer software product is stored in a storage medium, and includes several instructions for instructing a computer device (which may be a personal computer, a server, or a network device) to perform all or a part of the steps of the methods described in embodiments of this application. The storage medium includes: any medium that can store program code, such as a USB flash drive, a removable hard disk, a read-only memory (Read-Only Memory, ROM), a random access memory (Random Access Memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1. A data processing method, wherein the method comprises:

    obtaining a first single nucleotide polymorphism SNP set, wherein the first SNP set comprises N SNPs, and N is a positive integer;
    determining a second SNP set from the first SNP set based on first information of the SNP, wherein the first information comprises at least one of linkage disequilibrium information or a variance, the second SNP set comprises M SNPs, M is a positive integer, and M is less than N; and
    determining a third SNP set from the second SNP set based on a target feature extraction method and a non-linear feature extraction method, wherein the target feature extraction method comprises at least one of a filter feature extraction method, an envelope feature extraction method, or an embedded feature extraction method, the third SNP set comprises H SNPs, H is a positive integer, and H is less than M.

2. The method according to claim 1, wherein the first information comprises the linkage disequilibrium information, the linkage disequilibrium information comprises a linkage disequilibrium LD coefficient, and determining the second SNP set from the first SNP set based on the first information of the SNP comprises:
deleting a target SNP from the first SNP set based on the LD coefficient of the SNP to obtain the second SNP set, wherein the target SNP is an SNP whose LD coefficient is outside a preset LD coefficient interval.

3. The method according to claim 1 or 2, wherein the first information comprises the variance, and determining the second SNP set from the first SNP set based on the first information of the SNP comprises:
when there are a plurality of SNPs with a same variance in the first SNP set, retaining one SNP in the plurality of SNPs with the same variance, and deleting the other SNPs in the plurality of SNPs to obtain the second SNP set.

4. The method according to any one of claims 1 to 3, wherein determining the third SNP set from the second SNP set based on the target feature extraction method and the non-linear feature extraction method comprises:

   determining a fourth SNP set from the second SNP set based on the target feature extraction method, wherein the fourth SNP set comprises K SNPs, K is a positive integer, and K is less than M and greater than H; and
   determining the third SNP set from the fourth SNP set based on the non-linear feature extraction method.

5. The method according to claim 4, wherein determining the fourth SNP set from the second SNP set based on the target feature extraction method comprises:

   removing an SNP from the second SNP set based on the target feature extraction method; and
   when a coefficient of determination of remaining SNPs in the second SNP set is greater than a first threshold or a first remaining ratio is less than a first ratio threshold, determining the remaining SNPs in the second SNP set as the fourth SNP set, wherein the first remaining ratio is a ratio of a quantity of the remaining SNPs in the second SNP set to M.

6. The method according to claim 4 or 5, wherein determining the third SNP set from the fourth SNP set based on the non-linear feature extraction method comprises:

   removing an SNP from the fourth SNP set based on the non-linear feature extraction method; and
   when a coefficient of determination of remaining SNPs in the fourth SNP set is greater than a second threshold or a second remaining ratio is less than a second ratio threshold, determining the remaining SNPs in the fourth SNP set as the third SNP set, wherein the second remaining ratio is a ratio of a quantity of the remaining SNPs in the fourth SNP set to M.

7. A data processing apparatus, wherein the apparatus comprises a transceiver unit and a processing unit;

   the transceiver unit is configured to obtain a first SNP set, wherein the first SNP set comprises N SNPs, and N is a positive integer;
   the processing unit is configured to determine a second SNP set from the first SNP set based on first information of the SNP, wherein the first information comprises at least one of linkage disequilibrium information or a variance, the second SNP set comprises M SNPs, M is a positive integer, and M is less than N; and
   the processing unit is further configured to determine a third SNP set from the second SNP set based on a target feature extraction method and a non-linear feature extraction method, wherein the target feature extraction method comprises at least one of a filter feature extraction method, an envelope feature extraction method, or an embedded feature extraction method, the third SNP set comprises H SNPs, H is a positive integer, and H is less than M.

8. The apparatus according to claim 7, wherein the first information comprises the linkage disequilibrium information, the linkage disequilibrium information comprises a linkage disequilibrium LD coefficient, and the processing unit is specifically configured to:
delete a target SNP from the first SNP set based on the LD coefficient of the SNP to obtain the second SNP set, wherein the target SNP is an SNP whose LD coefficient is outside a preset LD coefficient interval.

9.  The apparatus according to claim 7 or 8, wherein the first information comprises the variance, and the processing unit is specifically configured to:
    when there are a plurality of SNPs with a same variance in the first SNP set, retain one SNP in the plurality of SNPs with the same variance, and delete the other SNPs in the plurality of SNPs to obtain the second SNP set.

10. The apparatus according to any one of claims 7 to 9, wherein the processing unit is specifically configured to:

    determine a fourth SNP set from the second SNP set based on the target feature extraction method, wherein the fourth SNP set comprises K SNPs, K is a positive integer, and K is less than M and greater than H; and
    determine the third SNP set from the fourth SNP set based on the non-linear feature extraction method.

11. The apparatus according to claim 10, wherein the processing unit is specifically configured to:

    remove an SNP from the second SNP set based on the target feature extraction method; and
    when a coefficient of determination of remaining SNPs in the second SNP set is greater than a first threshold or a first remaining ratio is less than a first ratio threshold, determine the remaining SNPs in the second SNP set as the fourth SNP set, wherein the first remaining ratio is a ratio of a quantity of the remaining SNPs in the second SNP set to M.

12. The apparatus according to claim 10 or 11, wherein the processing unit is specifically configured to:

    remove an SNP from the fourth SNP set based on the non-linear feature extraction method; and
    when a coefficient of determination of remaining SNPs in the fourth SNP set is greater than a second threshold or a second remaining ratio is less than a second ratio threshold, determine the remaining SNPs in the fourth SNP set as the third SNP set, wherein the second remaining ratio is a ratio of a quantity of the remaining SNPs in the fourth SNP set to M.

13. A data processing apparatus, comprising at least one processor and a memory, wherein the at least one processor executes a program or instructions stored in the memory, to enable the data processing apparatus to implement the method according to any one of claims 1 to 6.

14. A computer-readable storage medium, configured to store a computer program, wherein when the computer program is run on a computer or a processor, the computer or the processor is enabled to implement the method according to any one of claims 1 to 6.

15. A computer program product, wherein the computer program product comprises instructions, and when the instructions are run on a computer or a processor, the computer or the processor is enabled to implement the method according to any one of claims 1 to 6.

16. A chip, comprising at least one processor and a memory, wherein the at least one processor executes a program or instructions stored in the memory, to enable the data processing apparatus to implement the method according to any one of claims 1 to 6.

```
┌─────────────────────────────────────────────────────────┐
│              Data processing system 10                  │
│                                                         │
│   ┌─────────────────┐      ┌─────────────────┐         │
│   │  Communication  │      │   Processing    │         │
│   │   module 11     │      │   module 12     │         │
│   └─────────────────┘      └─────────────────┘         │
│                                                         │
│                                                         │
│   ┌─────────────────┐      ┌─────────────────┐         │
│   │    Storage      │      │   Interface     │         │
│   │   module 13     │      │   module 14     │         │
│   └─────────────────┘      └─────────────────┘         │
└─────────────────────────────────────────────────────────┘
```

FIG. 1

```
┌──────────────────────────────────────────────────────────┐
│                                                          │   S201
│              Obtain a first SNP set                      │
│                                                          │
└──────────────────────────────────────────────────────────┘
                            │
                            ▼
┌──────────────────────────────────────────────────────────┐
│  Determine a second SNP set from the first SNP set        │   S202
│  based on first information of an SNP, where the first     │
│  information includes at least one of linkage              │
│  disequilibrium information or a variance                  │
└──────────────────────────────────────────────────────────┘
                            │
                            ▼
┌──────────────────────────────────────────────────────────┐
│  Determine a third SNP set from the second SNP set         │
│  based on a target feature extraction method and a         │
│  non-linear feature extraction method, where the target    │   S203
│  feature extraction method includes at least one of a      │
│  filter feature extraction method, an envelope feature     │
│  extraction method, or an embedded feature extraction      │
│  method                                                    │
└──────────────────────────────────────────────────────────┘
```

FIG. 2

Data processing apparatus 300

Transceiver unit 301

Processing unit 302

FIG. 3

Chip 400

Processor 401

Bus 403

Interface circuit 402

FIG. 4

500

Electronic device

Processor

CPU 0

CPU 1    501

Processor

CPU 0

CPU 1    507

503    Memory 504

Transceiver    502

Output device    505

Input device    506

FIG. 5

Control circuit
603

Processor
601

Memory
602

Input/Output apparatus
604

FIG. 6

**INTERNATIONAL SEARCH REPORT**

| International application No. |
| --- |
| **PCT/CN2024/073782** |

**A. CLASSIFICATION OF SUBJECT MATTER**

G16B 40/00(2019.01)i; G06F 18/213(2023.01)i; G06F 16/9035(2019.01)i

According to International Patent Classification (IPC) or to both national classification and IPC

**B. FIELDS SEARCHED**

Minimum documentation searched (classification system followed by classification symbols)

IPC: G16B G06F

Documentation searched other than minimum documentation to the extent that such documents are included in the fields searched

Electronic data base consulted during the international search (name of data base and, where practicable, search terms used)

CNABS: CNTXT; CNKI; VEN; USTXT; WOTXT; EPTXT; IEEE: 华为, 单核苷酸多态性, 连锁不平衡, 方差, 过滤式, 包裹式, 嵌入式, 系数, 阈值, SNP, filter, wrapper, embedded, coefficient, threshold, linkage disequilibrium

**C. DOCUMENTS CONSIDERED TO BE RELEVANT**

| Category* | Citation of document, with indication, where appropriate, of the relevant passages | Relevant to claim No. |
| --- | --- | --- |
| X | CN 115019885 A (SICHUAN NEW HOPE LIUHE PIG BREEDING TECHNOLOGY CO., LTD. et al.) 06 September 2022 (2022-09-06) description, paragraphs [0054]-[0111] | 1-4, 7-10, 13-16 |
| A | CN 115019885 A (SICHUAN NEW HOPE LIUHE PIG BREEDING TECHNOLOGY CO., LTD. et al.) 06 September 2022 (2022-09-06) description, paragraphs [0054]-[0111] | 5, 6, 11, 12 |
| A | CN 112102880 A (NOVOGENE CO., LTD.) 18 December 2020 (2020-12-18) entire document | 1-16 |
| A | CN 113555063 A (ZHONGKAI UNIVERSITY OF AGRICULTURE AND ENGINEERING) 26 October 2021 (2021-10-26) entire document | 1-16 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **21 March 2024** | **01 April 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2024/073782** |

| Patent document cited in search report | | | Publication date (day/month/year) | Patent family member(s) | Publication date (day/month/year) |
| --- | --- | --- | --- | --- | --- |
| CN | 115019885 | A | 06 September 2022 | None | |
| CN | 112102880 | A | 18 December 2020 | None | |
| CN | 113555063 | A | 26 October 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 202310727647